# EUROPEAN PATENT SPECIFICATION

(11) **EP 3 536 291 B1**
(45) Date of publication and mention of the grant of the patent: **27.01.2021**
(21) Application number: 18290020.9
(22) Date of filing: 05.03.2018
(51) Int. Cl.: A61F 13/00, A61Q 1/14, A61K 8/02, D04H 1/02, D04H 1/425, A61F 13/12, A61F 13/45

(54) **A SKIN CARE PAD AND A METHOD FOR ITS MANUFACTURE**
HAUTPFLEGEKISSEN UND VERFAHREN ZU DESSEN HERSTELLUNG
TAMPON POUR LES SOINS DE LA PEAU ET SON PROCÉDÉ DE FABRICATION

(43) Date of publication of application: 11.09.2019
(73) Proprietor: Essity Operations France, 93400 Saint-Ouen (FR)
(72) Inventor: GREGOIRE, Philippe, 27700 LES ANDELYS (FR); RAMAMOORTHY, Karthik, 27400 LOUVIERS (FR)
(74) Representative: Essity Hygiene and Health AB

(56) References cited:
- WO-A1-98/16349
- WO-A1-98/57608
- WO-A1-2004/073757

## Description

### TECHNICAL FIELD

The disclosure pertains to a skin care pad comprising at least one layer of web material the skin care pad having a first main surface and a second main surface opposite to the first main surface, and having a first maximum extension along a first line extending in a length direction and a second maximum extension along a second line extending in a width direction, the first and second lines intersecting with each other and being perpendicular to each other and the second line constituting an axis of symmetry of the skin care pad, the skin care pad having a curvilinear peripheral edge. The disclosure also pertains to a method for cutting out skin care pads from a continuous web of web material.

### BACKGROUND

Skin care pads include articles which are used for application of make-up, facial cream, lotion, etc., as well as web articles which are used for make-up removal and skin cleansing. Most of the currently available skin care pads are in the form of generally flat circular, oval or square pads which are cut out from a web of pad material. They are often composed of a mixture of cotton fibers of various qualities or a mixture of cotton fibers and other fibers such as other natural fibers, regenerated fibers or synthetic fibers depending on the desired absorption and web properties for which the skin care pads are intended. Furthermore, skin care pads being made exclusively from artificial fibers such as regenerated fibers or synthetic fibers are also known in the art. It is further known to produce skin care pads from foam materials.

The skin care pads may have a substantially homogeneous composition of may be layered articles having different composition and/or different surface textures on the two major surfaces of the pad, as is disclosed in WO 01/42548 A1. A two-sided pad may provide a more versatile pad which may be used for different purposes, with one side being better adapted e.g. for skin cleansing and for the application of makeup, makeup remover, skin treatment products, etc., while the other side may be softer and optimized for web and absorbing excess products which have been applied to the skin.

Although two-sidedness of a skin care pad may contribute to increase the usefulness of the pad, there is still room for further improvement as it may be difficult e.g. to accurately apply makeup to a desired portion of the skin or to wipe away undesired make-up or other product without smudging correctly applied make-up.

In order to achieve accurate cosmetic application it is common to use particular cosmetic applicators, such as a cotton top or a specially designed applicator stick with a soft applicator tip. It has also been proposed in US2005/0257805 A1 to make small pads having the size of a fingertip and being provided with an adhesive backing for attaching the pad to a fingertip.

A further example of a disposable non-woven article for skin cleaning is disclosed in WO 98/16349 A1. The article has an elongate oval shape.

WO 2004/073757 A1 and WO 98/57608 A1 disclose absorbent articles for wearing in a user's crotch. The article in WO 2004/073757 A1 is a haemorrhoid treatment pad and the article in WO 98/57608 A1 is an interlabial pad.

The skin care pads as disclosed herein are disposable pads which are discarded after a single use. Hence, a further concern is to produce the skin care pads with a minimum of waste in an economical and simple manner.

An object is therefore to offer a skin care pad having improved versatility with regard to its usefulness for different wiping and product application purposes and which can be made in a cost-efficient, safe and simple manner and with a minimum of waste.

### SUMMARY

One or more of the above objects may be achieved with a make-up and cleansing pad in accordance with claim 1 and the method claim 13. Further embodiments are set out in the dependent claims, in the following description and in the drawings.

A skin care pad as disclosed herein comprises at least one layer of web material. The skin care pad has a first main surface and a second main surface opposite to the first main surface, and has a first maximum extension along a first line extending in a length direction and a second maximum extension along a second line extending in a width direction. The first and second lines intersect with each other and are perpendicular to each other. The second line constitutes an axis of symmetry of the skin care pad. The skin care pad has a peripheral edge. A ratio between the first maximum extension and the second maximum extension is 1.2-1.8, such as 1.25-1.6 or 1.3-1.4.

In a skin care pad as disclosed herein, the peripheral edge may has four convexely curved peak portions each convexely curved peak portion having an apex on one of the first line and the second line, whereby the convexely curved peak portions form a first pair of convexely curved peak portions being arranged opposite each other on the first line and a second pair of convexely curved peak portions being arranged opposite each other on the second line.

The convexely curved peak portions of the first pair of convexely curved peak portions may have a length along the peripheral edge of the skin care pad of from 13 to 30 mm, such as 15 to 20 mm, and may have a radius of curvature in the range of from 6 to 15 mm, such as from 7 to 12 mm.

As set out herein, one of the convexely curved peak portions of the second pair of convexely curved peak portions may have an extension along the peripheral edge which is shorter than an extension of the other convexely curved peak portion of the second pair of convexely curved peak portions. A ratio between a length of the shorter convexely curved peak portion of the second pair of convexely curved peak portions to the longer convexely curved peak portion of the second pair of convexely curved peak portions may be in the range of from 1/3 to 2/3, such as 1/2.

As set out herein, a radius of curvature of the convexely curved peak portions of the second pair of convexely curved peak portions may be in the range of from 8 mm to 25 mm, such as from 16 mm to 25 mm and wherein the radii of curvature of the convexely curved peak portions of the second pair of convexely curved peak portions may be the same or may differ from each other.

In a skin care pad wherein the convexely curved peak portions of the second pair of convexely curved peak portions have equal curvatures and extensions, the skin care pad may be symmetric also about the first line if the side edge portions of the peripheral edge of the skin care pad between the convexely curved portions of the peripheral edge have congruent shapes on each side of the first line.

In a skin care pad as disclosed herein, a radius of curvature of one or both of the convexely curved peak portions of the second pair of convexely curved peak portions is greater than a radius of curvature of the convexely curved peak portions of the first pair of convexely curved peak portions, such as from 1.5 to 5 times greater or 2 to 4 times greater.

As set out herein, the convexely curved portions of the first pair of convexely curved portions are preferably provided with a relatively sharp curvature creating a lobe at the edge of the skin care pad which lobe is sized such that it will accommodate a finger tip. Such finger tip sized lobes may be particularly suitable for makeup application and wiping in areas where a higher degree of accuracy is required, such as around the eyes. Portions of the pad having a less pronounced curvature may to advantage be used for purposes such as broad application of cream, cleansing products and for wiping of the brow, the cheeks, the décolletage, etc.

The peripheral edge of the skin care pad as disclosed herein, may comprise a pair of curved side edge portions, each side edge portion extending between one of the convexely curved peak portions of the first pair of convexely curved peak portions and one of the convexely curved peak portions of the second pair of convexely curved peak portions.

A radius of curvature of the convexely curved side edge portions of the peripheral edge of the skin care pad may be greater than a radius of curvature of any one of the curved peak portions and may be in the order of from 65 to 90 mm.

Accordingly, the skin care pad as disclosed herein may have a convexely curved side edge portion being arranged on either side of the second line, the convexely curved side edge portions being symmetric about the second line.

The peripheral edge of the skin care pad as disclosed herein, may comprise a pair of concavely curved side edge portions, each concavely curved side edge portion extending between one of the convexely curved peak portions of the first pair of convexely curved peak portions and one of the convexely curved peak portions of the second pair of convexely curved peak portions.

A radius of curvature of the concavely curved side edge portions of the peripheral edge of the skin care pad may be greater than a radius of curvature of any one of said curved peak portions and may be in the order of from 60 to 90 mm.

Accordingly, the skin care pad as disclosed herein may have a concavely curved side edge portion being arranged on either side of said second line, said concavely curved side edge portions being symmetric about said second line.

The skin care pad as disclosed herein may have a shape with convexely curved side edge portions connecting the convexely curved peak portions of the first pair of convexely curved peak portions and one or both of the convexely curved portions of the second pair of convexely curved peak portions. If all four convexely curved peak portions are connected by convexely curved side edge portions, the skin care pad may have an oval shape or a modified rhombic shape. If convexely curved side edge portions are arranged on only one side of the first line, the skin care pad will have an asymmetric shape, e.g. a shape resembling a rose petal or a gingko leaf.

The skin care pad as disclosed herein may have a shape with concavely curved side edge portions connecting the convexely curved peak portions of the first pair of convexely curved peak portions and one or both of the convexely curved peak portions of the second pair of convexely curved peak portions. If all four convexely curved peak portions are connected by concavely curved side edge portions, the skin care pad may have a shape resembling the outline of a lemon. If convexely curved side edge portions are arranged on only one side of the first line, the skin care pad will have an asymmetric shape.

In a skin care pad as disclosed herein, all side edge portions of the peripheral edge of the skin care pad which are arranged between the convexely curved peak portions may have the same shape and may include curved shapes and straight shapes and combinations thereof.

As set out herein, the skin care pad may comprise two convexely curved side edge portions which are symmetric about the second line and two concavely curved side edge portions which are also symmetric about the second line.

It has been found that a petal-shaped skin care pad having a pair of convexely curved side edge portions at each side of a first of the convexely curved peak portions of the second pair of convexely curved peak portions and a pair of concavely curved side edge portions at each side of the opposite, second convexely curved peak portion of the second pair of convexely curved peak portions, may be particularly useful as it conforms extremely well to the shape of a human hand and can be readily used for fine detail wiping and product application as well as for broad wiping and product application purposes. The petal-shaped skin care pad may be conveniently held between the four fingers of one hand and the thumb with the longitudinal direction of the skin care pad extending across the four fingers and the width direction of the skin care pad aligned with the extension of the fingers. The convexely curved side edge portions and the first convexely curved peak portion form a continuously convexely curved edge which is preferably placed at the finger tips and follows the contour of the finger tips. The concavely curved side edge portions and the second convexely curved peak portion are directed towards the wrist of the hand.

The skin care pad can be rotated in the hand to place one of the curved peak portions of the first pair of convexely curved peak portions at the tip of the index finger. In this way the convexely curved peak portion which is placed at the index finger can easily be used for wiping or product application where high precision is required, while the part of the skin care pad which is applied across the fingers of the user may be used for general wiping and product application.

In a skin care pad as disclosed herein, such as in a petal-shaped skin care pad, a notch or slit may be formed at one of the convexely curved peak portions of the second pair of convexely curved peak portions, the notch or slit extending across the skin care pad along the second line a distance corresponding to not more than 50% of a distance between the convexely curved portions of the second pair of convexely curved portions as measured along the second line. The notch or slit increases the flexibility of the skin care pad, and may thereby further improve its versatility for wiping and product application purposes.

The skin care pads as disclosed herein may be provided with one or more tear lines, such as a line of perforations, allowing the pad to be torn in two or more smaller parts along the tear line or tear lines. The tear line or tear lines are preferably arranged along the first line and/or the second line.

In a skin care pad having a notch or slit, the notch or slit may continue into a tear line, whereby the notch or slit will serve as a tear initiation means.

The web materials for use in the skin care pads as disclosed herein may comprise or consist of fibrous bonded or non-bonded materials. The fibers may be natural or man-made fibers and may be of a single kind through-out the web material or may be in the form of mixtures, layers or regions in the fibrous web. Natural fibers include cotton fibers, hemp, flax, etc. Man-made fibers may be regenerated fibers such as viscose, or synthetic fibers such as monocomponent, bicomponent or multicomponent polymeric fibers. The polymeric fibers may be thermally softenable or meltable to allow the fibers to be bonded together in a nonwoven web. Suitable polymers for the synthetic fibers are polyolefins, such as polyethylene and polypropylene, polyester (PET), nylon, etc.

The fibrous web materials may be any type of nonwoven material including carded webs, spunbond webs, meltblown webs, combinations of spunbond and meltblown webs, etc. The webs may be single layer webs or laminates of different materials and may be bonded by any suitable bonding method or combination of methods, such as thermal bonding, ultrasonic bonding, hydroentangling, needling, embossing, etc.

The web materials for use in the skin care pads as disclosed herein may comprise or consist of open-cell foam materials, made from absorbent or non-absorbent polymers. Fiber/foam laminates are also contemplated for the web materials of the skin care pads as disclosed herein.

Furthermore, the web materials for use in the skin care pads as disclosed herein may include a barrier layer, such as a skin layer on an open-cell foam material, or a polymeric film arranged at one surface of a fibrous or foam web material, or may be interposed between layers of fiber or foam.

In addition, the web material and/or the cut out skin care pads may be treated with a skin care product such as a cleansing agent, a moisturizer, a skin care cream, make-up, etc. The skin care product may be applied to all or part of the skin care pad and different products may be applied to different parts of the skin care pad. A "different part" of a skin care pad may be a different layer of the skin care pad or a different surface or part of a surface of the skin care pad.

The skin care pads as disclosed herein are generally two-dimensional articles having first and second opposing main surfaces. The first main surface of the skin care pad may have a surface texture and/or may comprise or consist of a material which is different from a surface texture and/or a material of the second main surface of the skin care pad.

A method of manufacturing the skin care pads as disclosed herein from a continuous web of pad material comprises:
- providing a continuous web of pad material;
- advancing the web of pad material in a machine direction, the machine direction being perpendicular to a cross direction of the web of pad material;
- cutting or punching out skin care pads from the web of pad material, the skin care pads having a first maximum extension along a first line extending in the machine direction and a second maximum extension along a second line extending in the cross machine direction, wherein the second line constitutes an axis of symmetry of the skin care pads and the skin care pads have a peripheral edge, the skin care pads being identical to each other and being arranged in at least 2 machine direction rows, such as 2-20 rows being aligned with a corresponding set of first lines, wherein the skin care pads of two adjacent machine direction rows are arranged in a staggered configuration both in the machine direction and in the cross machine direction.

The skin care pads of the method as disclosed herein have a ratio between the first maximum extension and the second maximum extension of the skin care pads is 1.2-1.8, such as 1.25-1.6 or 1.3-1.4 and are cut out with a smallest distance between pads of adjacent rows being from 3.6 mm to 6.5 mm, leaving a coherent reticulate rest web. The peripheral edge comprises four convexely curved peak portions each convexely curved peak portion having an apex on one of the first line and the second line, whereby the convexely curved peak portions form a first pair of convexely curved peak portions being arranged opposite each other on the first line and a second pair of convexely curved peak portions being arranged opposite each other on the second line, a radius of curvature of one or both of the convexely curved peak portions of the second pair of convexely curved peak portions being greater than a radius of curvature of the convexely curved peak portions of the first pair of convexely curved peak portions, such as from 1.5 to 5 times greater or 2 to 4 times greater,

The method as disclosed herein, allows the skin care pads to be produced in an efficient and economical manner with a minimum of waste. The reticulate rest web remaining after the skin care pads have been cut out and removed from the travelling continuous web has sufficient tensile strength to allow the rest web to be further processed in the continuously running production equipment without breaking or leaving small cut-away parts or trimmings which have to be removed by separate removal means.

The continuous web of pad material has a first and a second longitudinal edge and the rows of skin care pads include a corresponding first and second row arranged adjacent the first and second longitudinal edges. In the method as disclosed herein a smallest distance between each of the first and second longitudinal edges of the continuous web of pad material and the corresponding row of skin care pads may be in the range of from 5 mm to 7 mm. A selvage in this order on each side of the cut-out rows of skin care pads further contributes to improve the integrity and processability of the rest web.

The continuous web of pad material may be provided in the form of roll material or may be produced in line with the production of the skin care pads. As is conventional in the art, the continuous web of pad material has a uniform width with parallel side edges.

It may be preferred to produce the skin care pads by using a punching tool, whereby the punching tool is arranged to intermittently punch out two cross machine direction rows of skin care pads, the cross machine direction rows of skin care pads being separated in the machine direction such that the punching tool simultaneously cuts out skin care pads in a cross machine direction row n and a cross machine direction row n+3.

A punching method wherein the skin care pads are intermittently cut out from the continuous web of pad material in a pattern of cross machine direction rows being separated by two cross machine direction rows, such that a row n is cut out simultaneously with the row n+3, has been found particularly suitable for producing skin care pads with minimum waste while maintaining satisfactory integrity of the rest web for further processing in the production equipment.

### BRIEF DESCRIPTION OF THE DRAWINGS

The present invention will be further explained hereinafter by means of non-limiting examples and with reference to the appended drawings wherein:
- Fig. 1: shows a skin care pad as disclosed herein;
- Fig. 2: shows the skin care pad of Fig. 1 when held by a user;
- Fig. 3a-3d: show alternative outlines for a skin care pad as disclosed herein, the outline shown in Fig. 3c not being part of the claimed invention;
- Fig. 4: shows a production web for producing the skin care pads in Fig. 1; and
- Figs 5a-5c: show alternative productions webs for producing skin care pads as disclosed herein, the production web in Fig. 5c not being part of the claimed invention.

### DETAILED DESCRIPTION

The pads shown in the figures are provided as examples only and should not be considered limiting to the invention as disclosed herein. The skin care pads as disclosed herein may be made in different sizes and may be dry pads or wet pads. The surface area of skin care pads as disclosed herein typically range from about 2250 mm² in small size pads to about 6000 mm² for large pads. A medium sized skin care pad may have a surface area in the order of 3900 mm² to 4800 mm².

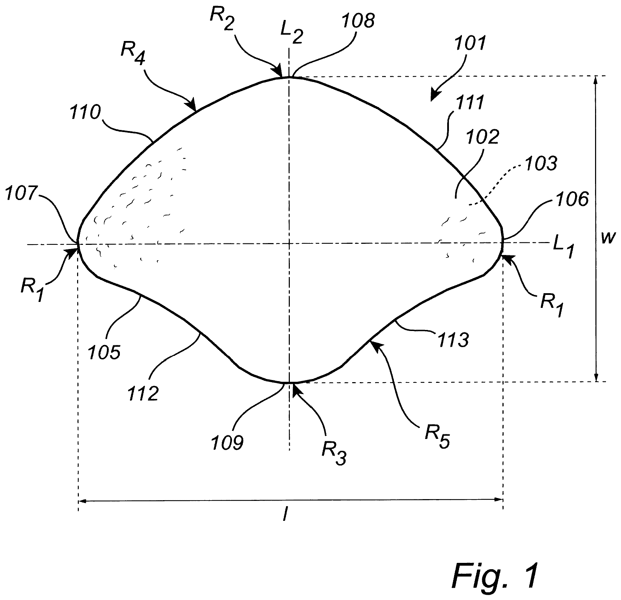

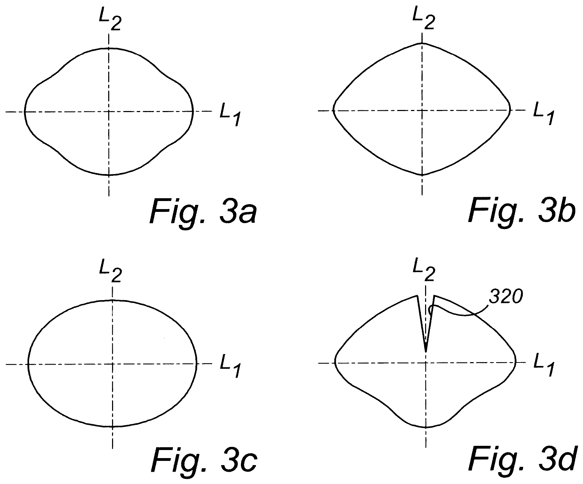

With reference to Fig. 1 and Figs. 3a-3d, the skin care pads 101, 301a, 301b, 301c and 301d may be made according to the method as disclosed herein by punching or cutting out the corresponding shapes from a travelling continuous web of material. The web material may be any kind of web material as disclosed herein, and may comprise fibers, foams, barrier materials and different kinds of additives such as skin care products (lotions, creams, make-up, skin cleansing agents, etc.), fragrances, colorants, superabsorbents, etc.

The skin care pad 101 comprises at least one layer of web material and has a generally two-dimensional appearance with a considerably smaller thickness than planar extension. The skin care pad 101 has a first main surface 102 and a second main surface 103 opposite to the first main surface 102. The skin care pad 101 has a first maximum extension or length I along a first line L₁ extending in a length direction and a second maximum extension or width w along a second line L₂ extending in a width direction. The first and second lines L₁, L₂ intersect with each other and are perpendicular to each other. As can be seen in Fig. 1, the second line L₂ constitutes an axis of symmetry of the skin care pad 101 as the two halves of the skin care pad 101 on either side of the second line L₂ have identical shape. The skin care pad 101 has a curvilinear peripheral edge 105 with different curvatures in the upper and lower halves of the skin care pad 101 as shown in Fig. 1 and is non-symmetrical about the first line L₁. The skin care pad 101 is longer than it is wide with a ratio between the length and the width being in the range of from 1.2-1.8, such as 1.25-1.6 or 1.3-1.4.

The peripheral edge 105 comprises four convexely curved peak portions 106, 107, 108, 109, each convexely curved peak portion having an apex on one of the first line L₁ and the second line L₂. Thereby, the convexely curved peak portions 106, 107, 108, 109 form a first pair of convexely curved peak portions 106, 107 which are arranged opposite each other on the first line L₁ and a second pair of convexely curved peak portions 108, 109 which are arranged opposite each other on the second line L₂.

The first pair of convexely curved peak portions 106, 107 have the same radius of curvature R₁. In the example shown in Fig. 1, the radius of curvature R₁ of the first pair of convexely curved peak portions 106, 107 is 10 mm.

In a skin care pad as disclosed herein a radius of curvature of the first pair of convexely curved peak portions being in the range of from 6 to 15 mm, such as from 7 to 12 mm has been found to be particularly well suited for creating a lobe which can accommodate a fingertip and be used for high precision wiping and product application. The length of the peripheral edge having such curvature may be in the range of from 13 to 30 mm, such as from 15 to 20 mm.

In the Fig. 1 skin care pad 101 also the second pair of convexely curved peak portions 108, 109 have the same radius of curvature R₂, R₃. As seen in Fig. 1, a first or upper convexely curved peak portion 108 of the second pair of convexely curved peak portions has a smaller extension along the peripheral edge 105 of the skin care pad 101 than the extension of the opposing second or lower convexely curved peak portion 109 of the second pair of convexely curved peak portions. In the example shown in Fig. 1, the radius of curvature R₂, R₃ of the second pair of convexely curved peak portions 108, 109 is 20 mm. However, the radius of curvature R₂, R₃ of the second pair of convexely curved peak portions 108, 109 may be in the range of from 8 mm to 25 mm, such as from 16 to 25 mm and the two convexely curved peak portions 108, 109 of the second pair of convexely curved peak portions 108, 109 may have different curvatures.

The first convexely curved peak portion 108 of the second pair of convexely curved peak portions 108, 109 may extend along a part of the peripheral edge 105 of the skin care pad 101 which is 1/3 to 2/3 of the extension of the second convexely curved peak portion 109 of the second pair of convexely curved peak portions 108, 109. In the example in Fig. 1, the first convexely curved peak portion 108 of the second pair of convexely curved peak portions 108, 109 has an extension along the peripheral edge 105 of the skin care pad 101 which is 1/2 of the extension of the second convexely curved peak portion 109 of the second pair of convexely curved peak portions 108, 109.

The skin care pad 101 in Fig. 1 has the general shape of a rose petal and is referred to herein as a "petal-shaped skin care pad". The petal-shaped skin care pad has a pair of convexely curved side edge portions 110, 111, one at each side of the first convexely curved peak portion 108 of the second pair of convexely curved peak portions 108, 109 and a pair of concavely curved side edge portions 112, 113, one at each side of the opposite, second convexely curved peak portion 109 of the second pair of convexely curved peak portions 108, 109.

The radius of curvature R₄ of the convexely curved side edge portions 110, 111 is considerably larger than the radii of curvature R₁, R₂, R₃ of any one of the convexely curved peak portions 106, 107, 108, 109 such as from 1.5 to 5 times larger or 2 to 4 times larger and may be in the order of from 65 mm to 90 mm. In the example shown in Fig. 1, the radius of curvature R₄ of the convexely curved side edge portions 110, 111 is 80 mm.

Also the radius of curvature R₅ of the concavely curved side edge portions 112, 113 is considerably larger than the radii of curvature R₁, R₂, R₃ of any one of the convexely curved peak portions 106, 107, 108, 109 and may be in the order of from 60 mm to 90 mm. In the example shown in Fig. 1, the radius of curvature R₄ of the convexely curved side edge portions 110, 111 is 70 mm.

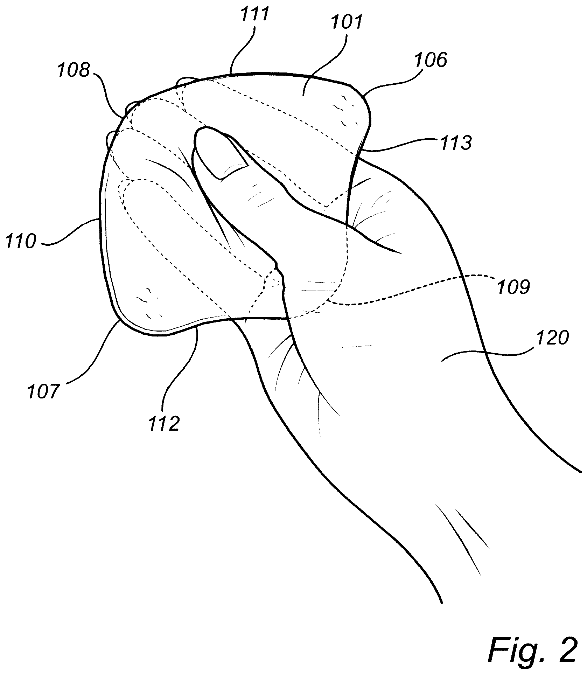

The petal-shaped skin care pad 101 conforms extremely well to the shape of a human hand 120 and may be conveniently held between the four fingers of one hand and the thumb as shown in Fig. 2. The skin care pad 101 is held with the longitudinal direction of the skin care pad extending across the four fingers and the width direction of the skin care pad aligned with the extension of the fingers. The convexely curved side edge portions 110, 111 and the first convexely curved peak portion 108 form a continuously convexely curved edge which follows the contour of the finger tips. The concavely curved side edge portions 112, 113 and the second convexely curved peak portion 109 are directed towards the wrist of the hand 120.

The first pair of convexely curved peak portions 106, 107 are placed at the sides of the fingers. The skin care pad 101 can easily be moved from the position which is shown in Fig. 2 by rotating it around the point where it is pinched between the thumb and the middle finger to bring e.g. the first convexely curved peak portions 106 of the first pair of convexely curved peak portions 106, 107 to the tip of the index finger. In this way the convexely curved peak portion 106 which is then placed at the index finger can be used for wiping or product application where high precision is required. When held as shown in Fig. 2, the skin care pad may be used for general wiping and product application. Shifting the skin care pad 101 between the different positions can be easily made using only the hand holding the skin care pad as it requires only normal dexterity.

With reference to Figs. 3a - 3d, there is shown a non-limiting number of alternative shapes of a skin care pad as disclosed herein. Fig. 3a shows a lemon-shaped outline for a skin care pad as disclosed herein. Fig. 3b shows a modified rhombic outline for a skin care pad as disclosed herein. The modified rhombic outline in Fig. 3b has four convexely curved peak portions forming rounded corners of the rhombic shape and four slightly convexely curved side edge portions connecting the convexely curved peak portions. It is to be understood that a modified rhombic shapes having straight or slightly concavely curved side edge portions are also contemplated for the skin care pads as disclosed herein. Fig. 3c shows an oval outline which does not form part of the claimed invention and Fig. 3d shows an outline similar to the outline of the skin care pad 101 in Fig. 1 but with a notch 320 arranged along the second line L₂ and extending from an upper part of the peripheral edge of the skin care pad as seen in Fig. 3d downwards towards the opposite part of the peripheral edge of the skin care pad. The notch 320 increases the flexibility of the skin care pad, and may further improve its versatility for wiping and product application purposes. The notch may take the form of a slit and may be applied to a skin care pad having any outline as disclosed herein.

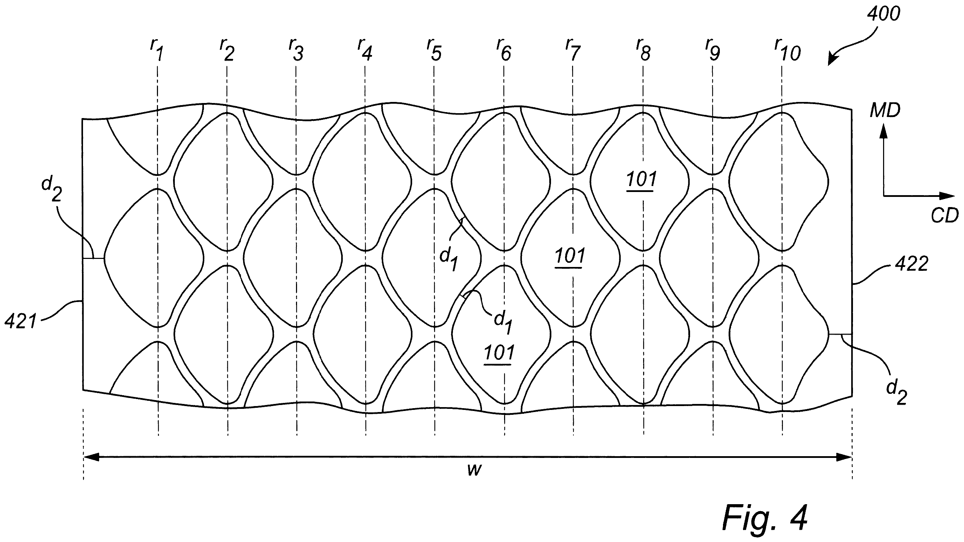

The skin care pads as disclosed herein may be made from a continuous web of pad material such as the continuous web of pad material 400 shown in Fig. 4.

The continuous web of pad material 400 is moved in a machine direction MD in a conventional way by means of rollers, transporters, etc. as known in the art. The machine direction MD is perpendicular to a cross machine direction CD of the web of pad material 400.

The web of pad material 400 is "endless" in a longitudinal direction corresponding to the machine direction MD and has a width W in the cross machine direction CD, the width W being measured between parallel side edges 421, 422 extending in the machine direction MD.

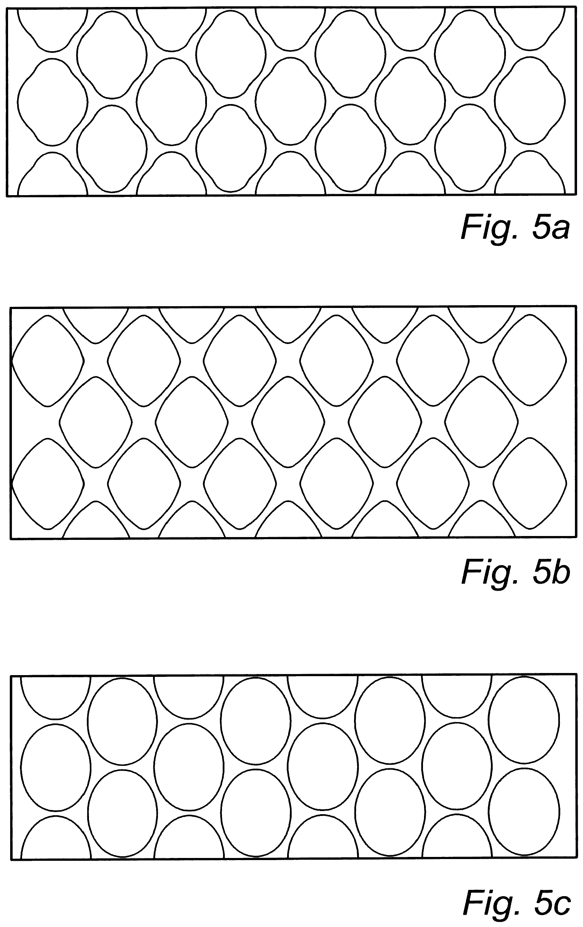

The skin care pads 101 are made by applying cutting or punching tools to the web of pad material to cut or punch out individual skin care pads having a desired shape. In the example shown in Fig. 4, the skin care pads 101 have the petal shape as shown in Figs. 1 and 2. Production webs for the manufacture of skin care pads having other shapes are shown in Figs. 5a-5c. The production web shown in Fig. 5c is not part of the claimed invention.

Referring to Fig. 1, the skin care pads 101 have a first maximum extension along a first line L₁ and a second maximum extension along a second line L₂. In the continuous web of pad material 400 in Fig. 4, the skin care pads 101 are arranged with the first maximum extension in the machine direction MD and the second maximum extension in the cross machine direction CD. All skin care pads 101 which are cut or punched out from the continuous web of pad material 400 are identical to each other and are arranged in a set of rows r₁ - r₁₀, with the first lines L₁ of the skin care pads 101 in each row being aligned with each other. The skin care pads 101 of two adjacent rows, such as the first row r₁ and the second row r₂ are arranged in a staggered configuration both in the machine direction MD and in the cross machine direction CD so that the individual skin care pads are closely packed and the edges of the skin care pads of each row are nested with the adjacent edges of the skin care pads of the adjacent row or rows. In the illustrated example, the number of rows of skin care pads is 10. However, it is to be understood that the number of rows r1-rₙ of skin care pads may be any suitable number, n, which can be accommodated in the continuous web of pad material 400, such as 2-20 rows.

As set out herein, the skin care pads 101 are longer than they are wide and a ratio between the first maximum extension i.e. the length and the second maximum extension i.e. the width of the skin care pads 101 is in the range of from 1.2-1.8, such as from 1.25-1.6 or 1.3-1.4.

The skin care pads 101 are cut out with a smallest distance d₁ between pads 101 of adjacent rows being in the range of from 3.6 mm to 6.5 mm. Thereby, the rest web which remains when the skin care pads 101 have been removed from the continuous web of pad material 400 forms a coherent reticulate web.

As set out above, the continuous web of pad material 400 has a first and a second longitudinal edge 421, 422 and the rows r₁-r₂₀ of skin care pads 101 include a corresponding first and second edge row r₁, r₂₀ which are arranged adjacent the first and second longitudinal edges 421, 422. A smallest distance d₂ between each of the first and second parallel longitudinal edge 421, 422 of the continuous web of pad material and the corresponding row r₁, r₂₀ of skin care pads 101 is in the range of from 5 mm to 7 mm.

The reticulate rest web remaining after the skin care pads 101 have been cut out and removed from the continuous web of pad material 400 has sufficient tensile strength to allow the rest web to be further processed in continuously running production equipment without breaking or leaving small cut-away parts or trimmings which have to be removed by separate removal means. A selvage on each side of the cut-out rows of skin care pads further contributes to improve the integrity and processability of the rest web.

The continuous web of pad material may be provided in the form of roll material or may be produced in line with the production of the skin care pads. As is conventional in the art, the continuous web of pad material has a uniform width with parallel side edges.

## Claims

1. A skin care pad (101) comprising at least one layer of web material said skin care pad (101) having a first main surface (102) and a second main surface (103) opposite to said first main surface (102), and having a first maximum extension along a first line (L₁) extending in a length direction and a second maximum extension along a second line (L₂) extending in a width direction, said first and second lines (L₁, L₂) intersecting with each other and being perpendicular to each other and said second line (L₂) constituting an axis of symmetry of said skin care pad (101), said skin care pad (101) having a peripheral edge (105), a ratio between said first maximum extension and said second maximum extension being in the range of from 1.2-1.8, such as 1.25-1.6 or 1.3-1.4, **characterized in that** said peripheral edge (105) comprises four convexely curved peak portions (106, 107, 108, 109) each convexely curved peak portion (106, 107, 108, 109) having an apex on one of said first line (L₁) and said second line (L₂), whereby said convexely curved peak portions (106, 107, 108, 109) form a first pair of convexely curved peak portions (106, 107) being arranged opposite each other on said first line (L₁) and a second pair of convexely curved peak portions (108, 109) being arranged opposite each other on said second line (L₂), a radius of curvature of one or both of the convexely curved peak portions of the second pair of convexely curved peak portions being greater than a radius of curvature of the convexely curved peak portions of the first pair of convexely curved peak portions, such as from 1.5 to 5 times greater or 2 to 4 times greater.

2. A skin care pad (101) according to claim 1, wherein said convexely curved peak portions (106, 107) of said first pair of convexely curved peak portions (106, 107) have a length of from 13 to 30 mm, such as from 15 to 20 mm.

3. A skin care pad (101) according to claim 2, wherein a radius of curvature (R₁) of said convexely curved peak portions (106, 107) of said first pair of convexely curved peak portions (106, 107) is in the range of from 6 to 15 mm, such as from 7 to 12 mm.

4. A skin care pad (101) according to any one of the preceding claims, wherein one of said convexely curved peak portions (108) of said second pair of convexely curved peak portions (108, 109) has an extension along said peripheral edge (105) of said skin care pad (101) which is shorter than an extension of said other convexely curved peak portion (109) of said second pair of convexely curved peak portions (108, 109), a ratio between a length of said shorter convexely curved peak portion (108) of said second pair of convexely curved peak portions (108, 109) to said longer convexely curved peak portion (109) of said second pair of convexely curved peak portions (108, 109) being in the range of from 1/3 to 2/3, such as 1/2.

5. A skin care pad (101) according to any one of the preceding claims, wherein a radius of curvature (R₂, R₃) of said convexely curved peak portions (108, 109) of said second pair of convexely curved peak portions (108, 109) is in the range of from 8 mm to 25 mm, such as from 16 to 25 mm, and wherein the radii of curvature (R₂, R₃) of said convexely curved peak portions (108, 109) of said second pair of convexely curved peak portions (108, 109) are the same or differ from each other.

6. A skin care pad (101) according to any one of the preceding claims, wherein said peripheral edge (105) comprises a pair of curved side edge portions (110, 111, 112, 113), each side edge portion (110, 111), extending between one of said convexely curved peak portions (106, 107) of said first pair of convexely curved peak portions (106, 107) and a first of said convexely curved peak portions (108, 109) of said second pair of convexely curved peak portions (108, 109).

7. A skin care pad (101) according to claim 6, wherein said side edge portions (110, 111) are convexely curved portions of said peripheral edge (105).

8. A skin care pad (101) according to claim 7, wherein said convexely curved side edge portions (110, 111) each have a radius of curvature (R₄) which is greater than a radius of curvature (R₁, R₂, R₃) of any one of said curved peak portions (106, 107, 108, 109).

9. A skin care pad (101) according to claim 6, wherein said side edge portions (112, 113) are concavely curved portions of said peripheral edge (105).

10. A skin care pad (101) according to claim 9, wherein said concavely curved side edge portions (112, 113) have a radius of curvature (R₅) which is greater than a radius of curvature (R₁, R₂, R₃) of any one of said curved peak portions (106, 107, 108, 109).

11. A skin care pad (101) according to any one of the preceding claims, wherein said skin care pad (101) comprises two convexely curved side edge portions (110, 111) which are symmetric about said second line (L₂) and two concavely curved side edge portions (112, 113) which are symmetric about said second line (L₂).

12. A skin care pad (101) according to any one of the preceding claims, wherein a radius of curvature (R₂, R₃) of one or both of said convexely curved peak portions (108, 109) of said second pair of convexely curved peak portions (108, 109) is greater than a radius of curvature (R₁) of said convexely curved peak portions (106, 107) of said first pair of convexely curved peak portions (106, 107), such as from 1.5 to 5 times greater or 2 to 4 times greater.

13. A method of manufacturing skin care pads (101) from a continuous web of pad material (400) said method comprising:
- providing a continuous web of pad material (400);
- advancing said web of pad material (400) in a machine direction (MD), said machine direction (MD) being perpendicular to a cross machine direction (CD) of said web of pad material (400);
- cutting or punching out skin care pads (101) from said web of pad material (400), said skin care pads (101) having a first maximum extension along a first line (L₁) extending in said machine direction (MD) and a second maximum extension along a second line (L₂) extending in said cross machine direction (CD), wherein said second line (L₂) constitutes an axis of symmetry of said skin care pads (101) and said skin care pads (101) have a peripheral edge (105), said skin care pads (101) being identical to each other and being arranged in at least 2 rows (r₁-rₙ), such as 2-20 rows aligned with a corresponding set of first lines (L₁), wherein said skin care pads (101) of two adjacent rows (r₁, r₂) are arranged in a staggered configuration both in said machine direction (MD) and in said cross machine direction (CD);
**characterized in that**
- a ratio between said first maximum extension and said second maximum extension of said skin care pads (101) is in the range of from 1.2-1.8, such as from 1.25-1.6 or 1.3-1.4;
- said peripheral edge (105) comprises four convexely curved peak portions (106, 107, 108, 109) each convexely curved peak portion (106, 107, 108, 109) having an apex on one of said first line (L₁) and said second line (L₂), whereby said convexely curved peak portions (106, 107, 108, 109) form a first pair of convexely curved peak portions (106, 107) being arranged opposite each other on said first line (L₁) and a second pair of convexely curved peak portions (108, 109) being arranged opposite each other on said second line (L₂), a radius of curvature of one or both of the convexely curved peak portions of the second pair of convexely curved peak portions being greater than a radius of curvature of the convexely curved peak portions of the first pair of convexely curved peak portions, such as from 1.5 to 5 times greater or 2 to 4 times greater; and
- said skin care pads (101) are cut out with a smallest distance (d₁) between pads (101) of adjacent rows being in the range of from 3.6 mm to 6.5 mm, leaving a coherent reticulate rest web.

14. A method according to claim 13, wherein said continuous web of pad material has a first and a second longitudinal edge (421, 422) and said rows (r₁-rₙ) of skin care pads (101) include a corresponding first and second edge row (r₁, rₙ) being arranged adjacent said first and second longitudinal edges (421, 422), a smallest distance (d₂) between each of said first and second parallel longitudinal edge (421, 422) of said continuous web of pad material and said corresponding row (r₁, rₙ) of skin care pads (101) being in the range of from 5 mm to 7 mm.

## Patentansprüche

1. Hautpflegetupfer (101), umfassend mindestens eine Schicht aus Bahnmaterial, wobei der Hautpflegetupfer (101) eine erste Hauptfläche (102) und eine zweite Hauptfläche (103) gegenüber der ersten Hauptfläche (102) aufweist und ein erstes Höchstausmaß entlang einer ersten Linie (L₁), die sich in einer Längsrichtung erstreckt, und ein zweites Höchstausmaß entlang einer zweiten Linie (L₂), die sich in einer Breitenrichtung erstreckt, aufweist, wobei die erste und zweite Linie (L₁, L₂) einander schneiden und senkrecht zueinander sind und die zweite Linie (L₂) eine Symmetrieachse des Hautpflegetupfers (101) beschreibt, wobei der Hautpflegetupfer (101) eine Außenkante (105) aufweist, wobei ein Verhältnis zwischen dem ersten Höchstausmaß und dem zweiten Höchstausmaß in dem Bereich von 1,2-1,8 ist, wie 1,25-1,6 oder 1,3-1,4, **dadurch gekennzeichnet, dass** die Außenkante (105) vier konvex gekrümmte Spitzenabschnitte (106, 107, 108, 109) umfasst, wobei jeder konvex gekrümmte Spitzenabschnitt (106, 107, 108, 109) einen Scheitelpunkt an einer der ersten Linie (L₁) und der zweiten Linie (L₂) aufweist, wodurch die konvex gekrümmten Abschnitte (106, 107, 108, 109) ein erstes Paar von konvex gekrümmten Spitzenabschnitten (106, 107), die gegenüber voneinander an der ersten Linie (L₁) eingerichtet sind, und ein zweites Paar von konvex gekrümmten Spitzenabschnitten (108, 109), die gegenüber voneinander an der zweiten Linie (L₂) eingerichtet sind, bilden, wobei ein Krümmungsradius eines oder beider der konvex gekrümmten Spitzenabschnitte des zweiten Paares von konvex gekrümmten Spitzenabschnitten größer ist als ein Krümmungsradius der konvex gekrümmten Spitzenabschnitte des ersten Paares von konvex gekrümmten Spitzenabschnitten, wie 1,5- bis 5-mal größer oder 2- bis 4-mal größer.

2. Hautpflegetupfer (101) nach Anspruch 1, wobei die konvex gekrümmten Spitzenabschnitte (106, 107) des ersten Paares von konvex gekrümmten Spitzenabschnitten (106, 107) eine Länge von 13 bis 30 mm aufweisen, wie 15 bis 20 mm.

3. Hautpflegetupfer (101) nach Anspruch 2, wobei ein Krümmungsradius (R₁) der konvex gekrümmten Spitzenabschnitte (106, 107) des ersten Paares von konvex gekrümmten Spitzenabschnitten (106, 107) in dem Bereich von 6 bis 15 mm ist, wie 7 bis 12 mm.

4. Hautpflegetupfer (101) nach einem der vorstehenden Ansprüche, wobei einer der konvex gekrümmten Spitzenabschnitte (108) des zweiten Paares von konvex gekrümmten Spitzenabschnitten (108, 109) ein Ausmaß entlang der Außenkante (105) des Hautpflegetupfers (101) aufweist, das kürzer als ein Ausmaß des anderen konvex gekrümmten Spitzenabschnitts (109) des zweiten Paares von konvex gekrümmten Spitzenabschnitten (108, 109) ist, wobei ein Verhältnis zwischen einer Länge des kürzeren konvex gekrümmten Spitzenabschnitts (108) des zweiten Paares von konvex gekrümmten Spitzenabschnitten (108, 109) zu dem längeren konvex gekrümmten Spitzenabschnitt (109) des zweiten Paares von konvex gekrümmten Spitzenabschnitten (108, 109) in dem Bereich von 1/3 bis 2/3 ist, wie 1/2.

5. Hautpflegetupfer (101) nach einem der vorstehenden Ansprüche, wobei ein Krümmungsradius (R₂, R₃) der konvex gekrümmten Spitzenabschnitte (108, 109) des zweiten Paares von konvex gekrümmten Spitzenabschnitten (108, 109) in dem Bereich von 8 mm bis 25 mm ist, wie 16 bis 25 mm, und wobei die Krümmungsradien (R₂, R₃) der konvex gekrümmten Spitzenabschnitte (108, 109) des zweiten Paares von konvex gekrümmten Spitzenabschnitten (108, 109) dieselben sind oder voneinander abweichen.

6. Hautpflegetupfer (101) nach einem der vorstehenden Ansprüche, wobei die Außenkante (105) ein Paar von gekrümmten Seitenkantenabschnitten (110, 111, 112, 113) umfasst, wobei sich jeder Seitenkantenabschnitt (110, 111) zwischen einem der konvex gekrümmten Spitzenabschnitte (106, 107) des ersten Paares von konvex gekrümmten Spitzenabschnitten (106, 107) und einem ersten der konvex gekrümmten Spitzenabschnitte (108, 109) des zweiten Paares von konvex gekrümmten Spitzenabschnitten (108, 109) erstreckt.

7. Hautpflegetupfer (101) nach Anspruch 6, wobei die Seitenkantenabschnitte (110, 111) konvex gekrümmte Abschnitte der Außenkante (105) sind.

8. Hautpflegetupfer (101) nach Anspruch 7, wobei die konvex gekrümmten Seitenkantenabschnitte (110, 111) jeweils einen Krümmungsradius (R₄) aufweisen, der größer als ein Krümmungsradius (R₁, R₂, R₃) eines beliebigen der gekrümmten Spitzenabschnitte (106, 107, 108, 109) ist.

9. Hautpflegetupfer (101) nach Anspruch 6, wobei die Seitenkantenabschnitte (112, 113) konkav gekrümmte Abschnitte der Außenkante (105) sind.

10. Hautpflegetupfer (101) nach Anspruch 9, wobei die konkav gekrümmten Seitenkantenabschnitte (112, 113) einen Krümmungsradius (R₅) aufweisen, der größer als ein Krümmungsradius (R₁, R₂, R₃) eines beliebigen der gekrümmten Spitzenabschnitte (106, 107, 108, 109) ist.

11. Hautpflegetupfer (101) nach einem der vorstehenden Ansprüche, wobei der Hautpflegetupfer (101) zwei konvex gekrümmte Seitenkantenabschnitte (110, 111), die um die zweite Linie (L₂) symmetrisch sind, und zwei konkav gekrümmte Seitenkantenabschnitte (112, 113), die um die zweite Linie (L₂) symmetrisch sind, umfasst.

12. Hautpflegetupfer (101) nach einem der vorstehenden Ansprüche, wobei ein Krümmungsradius (R₂, R₃) eines oder beider der konvex gekrümmten Spitzenabschnitte (108, 109) des zweiten Paares von konvex gekrümmten Spitzenabschnitten (108, 109) größer als ein Krümmungsradius (R₁) der konvex gekrümmten Spitzenabschnitte (106, 107) des ersten Paares von konvex gekrümmten Spitzenabschnitten (106, 107) ist, wie 1,5 bis 5-mal größer oder 2- bis 4-mal größer.

13. Verfahren zur Herstellung von Hautpflegetupfern (101) aus einer fortlaufenden Bahn von Tupfermaterial (400), wobei das Verfahren umfasst:
- Bereitstellen einer fortlaufenden Bahn von Tupfermaterial (400);
- Fortbewegen der Bahn von Tupfermaterial (400) in einer Maschinenrichtung (MD), wobei die Maschinenrichtung (MD) senkrecht zu einer Quermaschinenrichtung (CD) der Bahn von Tupfermaterial (400) ist;
- Ausschneiden oder Ausstanzen von Hautpflegetupfern (101) aus der Bahn von Tupfermaterial (400), wobei die Hautpflegetupfer (101) ein erstes Höchstausmaß entlang einer ersten Linie (L₁), die sich in der Maschinenrichtung (MD) erstreckt, und ein zweites Höchstausmaß entlang einer zweiten Linie (L₂), die sich in der Quermaschinenrichtung (CD) erstreckt, aufweist, wobei die zweite Linie (L₂) eine Symmetrieachse der Hautpflegetupfer (101) beschreibt und die Hautpflegetupfer (101) eine Außenkante (105) aufweisen, wobei die Hautpflegetupfer (101) identisch miteinander sind und in mindestens 2 Reihen (r₁-rₙ) eingerichtet sind, wie 2-20 Reihen, die mit einem entsprechenden Satz erster Linien (L₁) ausgerichtet sind, wobei die Hautpflegetupfer (101) zwei angrenzender Reihen (r₁, r₂) in einer versetzten Konfiguration, sowohl in der Maschinenrichtung (MD) als auch in der Quermaschinenrichtung (CD) eingerichtet sind,
**dadurch gekennzeichnet, dass**
- ein Verhältnis zwischen dem ersten Höchstausmaß und dem zweiten Höchstausmaß der Hautpflegetupfer (101) in dem Bereich von 1,2-1,8 ist, wie 1,25-1,6 oder 1,3-1,4;
- die Außenkante (105) vier konvex gekrümmte Spitzenabschnitte (106, 107, 108, 109) umfasst, wobei jeder konvex gekrümmte Spitzenabschnitt (106, 107, 108, 109) einen Scheitelpunkt auf einer der ersten Linie (L₁) und der zweiten Linie (L₂) aufweist, wodurch die konvex gekrümmten Spitzenabschnitte (106, 107, 108, 109) ein erstes Paar von konvex gekrümmten Spitzenabschnitten (106, 107), die gegenüber voneinander an der ersten Linie (L₁) eingerichtet sind, und ein zweites Paar von konvex gekrümmten Spitzenabschnitten (108, 109), die gegenüber voneinander an der zweiten Linie (L₂) eingerichtet sind, bilden, wobei ein Krümmungsradius eines oder beider der konvex gekrümmten Spitzenabschnitte des zweiten Paares von konvex gekrümmten Spitzenabschnitten größer als ein Krümmungsradius der konvex gekrümmten Spitzenabschnitte des ersten Paares von konvex gekrümmten Spitzenabschnitten ist, wie 1,5- bis 5-mal größer oder 2- bis 4-mal größer; und
- die Hautpflegetupfer (101) mit einem kleinsten Abstand (d₁) zwischen Tupfern (101) aus angrenzenden Reihen in dem Bereich von 3,6 mm bis 6,5 mm ausgeschnitten werden, wobei eine zusammenhängende netzähnliche Restbahn verbleibt.

14. Verfahren nach Anspruch 13, wobei die fortlaufende Bahn von Tupfermaterial eine erste und zweite Längskante (421, 422) aufweist und die Reihen (r₁-rₙ) von Hautpflegetupfern (101) eine entsprechende erste und zweite Kantenreihe (r₁, rₙ) beinhalten, die angrenzend an die erste und zweite Längskante (421, 422) eingerichtet sind, wobei ein kleinster Abstand (d₂) zwischen jeder der ersten und zweiten parallelen Längskante (421, 422) der fortlaufenden Bahn von Tupfermaterial und der entsprechenden Reihe (r₁, r₂) von Hautpflegetupfern (101) in dem Bereich von 5 mm bis 7 mm ist.

## Revendications

1. Tampon pour les soins de la peau (101) comprenant au moins une couche de matériau en bande, ledit tampon pour les soins de la peau (101) présentant une première surface principale (102) et une seconde surface principale (103) opposée à ladite première surface principale (102), et présentant une première extension maximale le long d'une première ligne (L₁) s'étendant dans une direction de la longueur et une seconde extension maximale le long d'une seconde ligne (L₂) s'étendant dans une direction de la largeur, lesdites première et seconde lignes (L₁, L₂) se croisent entre elles et étant perpendiculaires entre elles et ladite seconde ligne (L₂) constituant un axe de symétrie dudit tampon pour les soins de la peau (101), ledit tampon pour les soins de la peau (101) présentant un bord périphérique (105), un rapport entre ladite première extension maximale et ladite seconde extension maximale se situant dans la plage allant de 1,2 à 1,8, tel que de 1,25 à 1,6 ou de 1,3 à 1,4, **caractérisé en ce que** ledit bord périphérique (105) comprend quatre parties de crête incurvées de manière convexe (106, 107, 108, 109), chaque partie de crête incurvée de manière convexe (106, 107, 108, 109) présentant un sommet sur l'une parmi ladite première ligne (L₁) et ladite seconde ligne (L₂), moyennant quoi lesdites parties de crête incurvées de manière convexe (106, 107, 108, 109) forment une première paire de parties de crête incurvées de manière convexe (106, 107) étant disposées de manière opposée l'une à l'autre sur ladite première ligne (L₁) et une seconde paire de parties de crête incurvées de manière convexe (108, 109) étant disposées de manière opposée l'une à l'autre sur ladite seconde ligne (L₂), un rayon de courbure de l'une ou des deux parties de crête incurvées de manière convexe de la seconde paire de parties de crête incurvées de manière convexe étant supérieur à un rayon de courbure des parties de crête incurvées de manière convexe de la première paire de parties de crête incurvées de manière convexe, tel que de 1,5 à 5 fois supérieure ou de 2 à 4 fois supérieure.

2. Tampon pour les soins de la peau (101) selon la revendication 1, dans lequel lesdites parties de crête incurvées de manière convexe (106, 107) de ladite première paire de parties de crête incurvées de manière convexe (106, 107) présentent une longueur allant de 13 à 30 mm, telle que de 15 à 20 mm.

3. Tampon pour les soins de la peau (101) selon la revendication 2, dans lequel un rayon de courbure (R₁) desdites parties de crête incurvées de manière convexe (106, 107) de ladite première paire de parties de crête incurvées de manière convexe (106, 107) se situe dans la plage allant de 6 à 15 mm, tel que de 7 à 12 mm.

4. Tampon pour les soins de la peau (101) selon l'une quelconque des revendications précédentes, dans lequel l'une desdites parties de crête incurvées de manière convexe (108) de ladite seconde paire de parties de crête incurvées de manière convexe (108, 109) présente une extension le long dudit bord périphérique (105) dudit tampon pour les soins de la peau (101) qui est plus court qu'une extension de ladite autre partie de crête incurvée de manière convexe (109) de ladite seconde paire de parties de crête incurvées de manière convexe (108, 109), un rapport entre une longueur de ladite partie de crête incurvée de manière convexe plus courte (108) de ladite seconde paire de parties de crête incurvées de manière convexe (108, 109) et ladite partie de crête incurvée de manière convexe plus longue (109) de ladite seconde paire de parties de crête incurvées de manière convexe (108, 109) se situant dans la plage allant de 1/3 à 2/3, tel que 1/2.

5. Tampon pour les soins de la peau (101) selon l'une quelconque des revendications précédentes, dans lequel un rayon de courbure (R₂, R₃) desdites parties de crête incurvées de manière convexe (108, 109) de ladite seconde paire de parties de crête incurvées de manière convexe (108, 109) se situe dans la plage allant de 8 mm à 25 mm, tel que de 16 à 25 mm, et dans lequel les rayons de courbure (R₂, R₃) desdites parties de crête incurvées de manière convexe (108, 109) de ladite seconde paire de parties de crête incurvées de manière convexe (108, 109) sont identiques ou différents les uns des autres.

6. Tampon pour les soins de la peau (101) selon l'une quelconque des revendications précédentes, dans lequel ledit bord périphérique (105) comprend une paire de parties de bords latéraux incurvées (110, 111, 112, 113), chaque partie de bord latéral (110, 111), s'étendant entre l'une desdites parties de crête incurvées de manière convexe (106, 107) de ladite première paire de parties de crête incurvées de manière convexe (106, 107) et une première desdites parties de crête incurvées de manière convexe (108, 109) de ladite seconde paire de parties de crête incurvées de manière convexe (108, 109).

7. Tampon pour les soins de la peau (101) selon la revendication 6, dans lequel lesdites parties de bords latéraux (110, 111) sont des parties incurvées de manière convexe dudit bord périphérique (105).

8. Tampon pour les soins de la peau (101) selon la revendication 7, dans lequel lesdites parties de bords latéraux incurvées de manière convexe (110, 111) présentent chacune un rayon de courbure (R₄) qui est supérieur à un rayon de courbure (R₁, R₂, R₃) de l'une quelconque des parties de crête incurvées (106, 107, 108, 109).

9. Tampon pour les soins de la peau (101) selon la revendication 6, dans lequel lesdites parties de bords latéraux (112, 113) sont des parties incurvées de manière concave dudit bord périphérique (105).

10. Tampon pour les soins de la peau (101) selon la revendication 9, dans lequel lesdites parties de bords latéraux incurvées de manière concave (112, 113) présentent un rayon de courbure (R₅) qui est supérieur à un rayon de courbure (R₁, R₂, R₃) de l'une quelconque desdites parties de crête incurvées (106, 107, 108, 109).

11. Tampon pour les soins de la peau (101) selon l'une quelconque des revendications précédentes, dans lequel ledit tampon pour les soins de la peau (101) comprend deux parties de bords latéraux incurvées de manière convexe (110, 111) qui sont symétriques autour de ladite seconde ligne (L₂) et deux parties de bords latéraux incurvées de manière concave (112, 113) qui sont symétriques autour de ladite seconde ligne (L₂).

12. Tampon pour les soins de la peau (101) selon l'une quelconque des revendications précédentes, dans lequel un rayon de courbure (R₂, R₃) de l'une ou deux desdites parties de crête incurvées de manière convexe (108, 109) de ladite seconde paire de parties de crête incurvées de manière convexe (108, 109) est supérieur à un rayon de courbure (R₁) desdites parties de crête incurvées de manière convexe (106, 107) de ladite première paire de parties de crête incurvées de manière convexe (106, 107), tel que de 1,5 à 5 fois supérieure ou de 2 à 4 fois supérieure.

13. Procédé de fabrication de tampons pour les soins de la peau (101) à partir d'une bande continue de matériau de tampon (400), ledit procédé comprenant :
- la fourniture d'une bande continue de matériau de tampon (400) ;
- l'avancée de ladite bande de matériau de tampon (400) en direction d'une machine (MD), ladite direction de la machine (MD) étant perpendiculaire à une direction transversale de la machine (CD) de ladite bande de matériau de tampon (400) ;
- le coupage ou le découpage de tampons pour les soins de la peau (101) de ladite bande de matériau de tampon (400), lesdits tampons pour les soins de la peau (101) présentant une première extension maximale le long d'une première ligne (L₁) s'étendant dans ladite direction de la machine (MD) et une seconde extension maximale le long d'une seconde ligne (L₂) s'étendant dans ladite direction transversale de la machine (CD), dans lequel ladite seconde ligne (L₂) constitue un axe de symétrie desdits tampons pour les soins de la peau (101) et lesdits tampons pour les soins de la peau (101) présentent un bord périphérique (105), lesdits tampons pour les soins de la peau (101) étant identiques les uns aux autres et étant disposés en au moins deux rangées (ri à rₙ), telles que de 2 à 20 rangées alignées avec une série correspondante de premières lignes (L₁), dans lequel lesdits tampons pour soins de la peau (101) de deux rangées adjacentes (r₁, r₂) sont disposés selon une configuration en quinconce à la fois dans ladite direction de la machine (MD) et dans ladite direction transversale de la machine (CD) ;
**caractérisé en ce que**
- un rapport entre ladite première extension maximale et ladite seconde extension maximale desdits tampons pour les soins de la peau (101) se situe dans la plage allant de 1,2 à 1,8, tel que de 1,25 à 1,6 ou de 1,3 à 1,4 ;
- ledit bord périphérique (105) comprend quatre parties de crête incurvées de manière convexe (106, 107, 108, 109), chaque partie de crête incurvée de manière convexe (106, 107, 108, 109) présentant un sommet sur l'une parmi ladite première ligne (L₁) et ladite seconde ligne (L₂), moyennant quoi lesdites parties de crête incurvées de manière convexe (106, 107, 108, 109) forment une première paire de parties de crête incurvées de manière convexe (106, 107) étant disposées de manière opposée l'une à l'autre sur ladite première ligne (L₁) et une seconde paire de parties de crête incurvées de manière convexe (108, 109) étant disposées de manière opposée l'une à l'autre sur ladite seconde ligne (L₂), un rayon de courbure de l'une ou des deux parties de crête incurvées de manière convexe de la seconde paire de parties de crête incurvées de manière convexe étant supérieur à un rayon de courbure des parties de crête incurvées de manière convexe de la première paire de parties de crête incurvées de manière convexe, tel que de 1,5 à 5 fois supérieure ou de 2 à 4 fois supérieure ; et
- lesdits tampons pour les soins de la peau (101) sont coupés avec une plus petite distance (d₁) entre les tampons (101) des rangées adjacentes se situant dans la plage allant de 3,6 mm à 6,5 mm, laissant une bande résiduelle réticulée homogène.

14. Procédé selon la revendication 13, dans lequel ladite bande continue de matériau de tampon présente un premier et un second bords périphériques (421, 422) et lesdites rangées (r₁ à rₙ) des tampons pour les soins de la peau (101) incluent une première et une seconde rangées de bords correspondantes (r₁, rₙ) étant disposées de manière adjacente auxdits premier et second bords longitudinaux (421, 422), une plus petite distance (d₂) entre chacun desdits premier et second bords longitudinaux parallèles (421, 422) de ladite bande continue de matériau de tampon et ladite rangée correspondante (r₁, rₙ) des tampons pour les soins de la peau (101) se situant dans la plage allant de 5 mm à 7 mm.
